# FASCICULE DE BREVET EUROPEEN

(11) **EP 0 948 310 B1**
(45) Date de publication et mention de la délivrance du brevet: **11.12.2002**
(21) Numéro de dépôt: 97912263.7
(22) Date de dépôt: 27.10.1997
(51) Int. Cl.: A61K 7/06

(54) **COMPOSITION COSMETIQUE COMPRENANT UN POLYMERE ANIONIQUE OU NON IONIQUE ET UNE SILICONE CARBOXYLIQUE**
KOSMETISCHE ZUSAMMENSETZUNG, DIE EIN ANIONISCHES ODER NICHTIONISCHES POLYMER UND MINDESTENS EIN CARBOXYSILIKON ENTHÄLT.
COSMETIC COMPOSITION CONTAINING AN ANIONIC OR NON IONIC POLYMER AND A CARBOXYLIC SILICON

(30) Priorité: 12.11.1996 FR 9613759
(43) Date de publication de la demande: 13.10.1999
(73) Titulaire: L'OREAL, 75008 Paris (FR)
(72) Inventeur: DUPUIS, Christine, F-75018 Paris (FR)
(74) Mandataire: Miszputen, Laurent
(86) Numéro de dépôt international: FR9701922
(87) Numéro de publication internationale: WO98020833

(56) Documents cités:
- WO-A-92/21316
- WO-A-95/00108
- WO-A-95/23579

## Description

La présente invention a trait à une composition cosmétique pour le traitement des fibres kératiniques, en particulier des cheveux, comprenant au moins un polymère anionique ou non ionique et au moins une silicone comprenant au moins une fonction acide carboxylique , ainsi qu'au procédé de traitement des fibres kératiniques à l'aide de cette composition.
Les compositions de maintien ou de mise en forme des cheveux contenant dans leur formulation des polymères de coiffage (polymères fixants) présentent généralement l'inconvénient de rendre difficile le démêlage, le recoiffage ou le brossage des cheveux, en particulier pendant un brushing. Les cheveux traités avec ces polymères fixants sont généralement rêches et ont un toucher non naturel.

L'association de dérivés siliconés avec des polymères fixants est connue dans des compositions cosmétiques pour le maintien et/ou la fixation de la coiffure. Il a été constaté que ces dérivés siliconés peuvent améliorer les propriétés de démêlage, de douceur et de brillance des cheveux traités à l'aide de ces compositions. Cependant, les dérivés siliconés ne sont pas favorables aux propriétés coiffantes des compositions contenant des polymères fixants et les propriétés de démêlage ou de douceur ne sont pas encore satisfaisantes.

Le but de la présente invention est donc de proposer des compositions permettant de fixer et/ou de mettre en forme la coiffure, ces compositions devant avoir de bonnes propriétés de fixation et/ou de tenue dans le temps et apporter d'excellentes propriétés cosmétiques telles que la douceur, le démêlage et le toucher.
On connaît par le document EP-A-0 219 830 des compositions capillaires contenant un polymère cationique et un polydiméthylsiloxane à fonction carboxylique. Cependant, l'addition du polydiméthylsiloxane n'améliore pas les propriétés de douceur des cheveux traités avec un polymère cationique.
WO95/00108 décrit des compositions capillaires liquides contenant un polymère siliconé à squelette polyvinylique et à greffons fluorés fixant, associé à une résine non siliconée fixante. WO92/21316 décrit des compositions capillaires contenant une silicone, un latex, un agent de suspension du latex et de la silicone et/ou un agent épaississant.
Or, la demanderesse a découvert de manière surprenante qu'en utilisant des compositions contenant un polymère anionique ou non ionique en association avec au moins une silicone comprenant au moins une fonction acide carboxylique dans un milieu cosmétiquement acceptable, on obtient d'excellentes propriétés cosmétiques telles que la douceur, le démêlage et le toucher tout en ayant d'excellentes propriétés coiffantes et/ou fixantes.

La présente invention a donc pour objet une composition cosmétique, en particulier pour les fibres kératiniques comprenant, dans un milieu cosmétiquement acceptable, au moins un polymère anionique ou non ionique et au moins une silicone comprenant au moins une fonction acide carboxylique.

Dans le cadre de la présente demande, on entend par compositions cosmétiques pour le maintien de la coiffure toute composition ayant pour fonction de fixer temporairement la forme de la coiffure, comme par exemple les laques et sprays de coiffage, les gels et les mousses de coiffage. Par pouvoir fixant de la composition, on désigne l'aptitude de cette dernière à donner aux cheveux une cohésion de telle sorte que la mise en forme initiale de la coiffure soit conservée. Par polymère fixant, on entend tout polymère ayant pour fonction de fixer la forme de la coiffure.

Selon la présente invention, par fibres kératiniques, on comprend les cheveux, les cils, les sourcils et plus particulièrement les cheveux.

Selon la présente invention, par groupement acide carboxylique, on comprend la forme acide libre, la forme neutralisée par une base et leurs mélanges. Selon l'invention, le groupement acide carboxylique ne résulte pas de la polymérisation d'un ou plusieurs monomères anioniques à insaturation éthylénique.

Dans tout ce qui suit ou qui précède, on entend désigner par silicone, en conformité avec l'acception générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles notamment en C₁-C₁₀ et en particulier méthyle, les radicaux fluoroalkyles, les radicaux aryles et en particulier phényle.

La silicone comprenant au moins un groupement acide carboxylique peut être un organopolysiloxane comprenant au moins un motif : 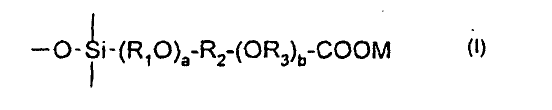 dans lequel R₁ et R₃ désignent indépendamment un radical alkylène linéaire ou ramifié ayant de 2 à 20 atomes de carbone, R₂ désigne un radical alkylène linéaire ou ramifié ayant de 1 à 50 atomes de carbone pouvant comprendre un groupement hydroxyle,
a représente 0 ou 1 et b est un nombre allant de 0 à 200, M désigne hydrogène, un métal alcalin ou alcalino-terreux, NH₄, un groupement ammonium quaternaire tel qu'un groupement mono-, di-, tri- ou tétra (alkyl C₁₋C₄) ammonium.
R₁ et R₃ peuvent par exemple désigner éthylène, propyléne, butylène.

On peut par exemple utiliser les organopolysiloxanes à groupements carboxyliques de formule (II) : 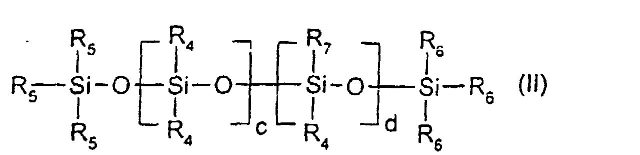 dans laquelle les radicaux R4 sont identiques ou différents les uns des autres et sont choisis parmi un radical alkyle en C₁-C₂₂, linéaire ou ramifié, un radical alcoxy en C₁-C₂₂ et un radical phényle,
les radicaux R₅, R₆, R₇ sont identiques ou différents les uns des autres et sont choisis parmi un radical alkyle en C₁-C₂₂, linéaire ou ramifié, un radical alcoxy en C₁-C_{22,} un radical phényle et un radical -(R₁O)ₐ-R₂-(OR₃)_{b}-COOM,
au moins l'un des radicaux R₅, R₆, R₇ étant un radical -(R₁O)ₐ-R₂-(OR₃)_{b}-COOM, les radicaux R₁, R₂, R₃, a, b et M ayant la même signification que ci-dessus,
c et d sont des nombres allant de 0 à 1000, la somme c+d allant de préférence de 2 à 1000.

Parmi les silicones de formule (II), on préfère les composés de formule (III) suivante : 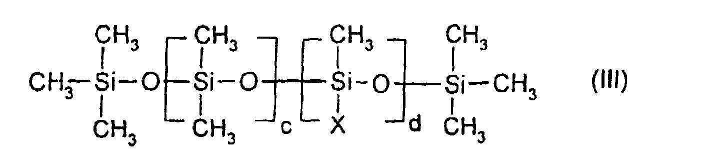 dans laquelle X est un radical -(R₁O)ₐ-R₂-(OR₃)_{b}-COOM. les radicaux R₁, R₂, R₃, a, b, d et M ayant la même signification que ci-dessus.

Encore plus particulièrement, on préfère les composés de formule (III) dans laquelle a et b sont égaux à 0 et R₂ est un groupement alkylène, linéaire ou ramifié, en C₂-C₁₂ tel que (CH₂)₉, (CH₂)₁₀ ou -CH(CH₃)-

Les silicones comprenant au moins un groupement acide carboxylique sont notamment décrites dans les demandes de brevets WO95/23579 et EP-A-0 219 830.

Des composés répondant à la formule (III) ci-dessus sont par exemple commercialisés sous la dénomination Huile M 642 par la société WACKER, sous les dénominations SLM 23 000/1, SLM 23 000/2 par la société WACKER sous la dénomination 176-12057 par la société GENERAL ELECTRIC, sous la dénomination FZ 3703 par la société OSI, sous la dénomination BY 16 880 par la société TORAY SILICONE.

Selon l'invention, on peut utiliser tout polymère fixant anionique ou non ionique connu en soi.
Ces polymères fixants peuvent être utilisés sous forme solubilisée ou sous forme de dispersions de particules solides de polymère.

Les polymères fixants anioniques généralement utilisés sont des polymères comportant des groupements dérivés d'acide carboxylique, sulfonique ou phosphorique et ont un poids moléculaire moyen en poids compris entre environ 500 et 5.000.000.
1) Les groupements carboxyliques sont apportés par des monomères mono ou diacides carboxyliques insaturés tels que ceux répondant à la formule : 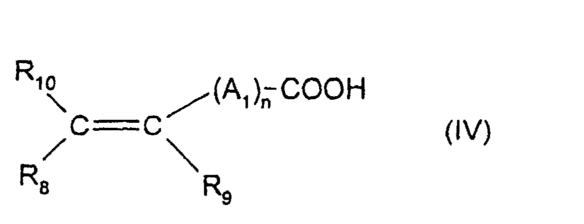
dans laquelle n est un nombre entier de 0 à 10, A₁ désigne un groupement méthylène, éventuellement relié à l'atome de carbone du groupement insaturé ou au groupement méthylène voisin lorsque n est supérieur à 1 par l'intermédiaire d'un hétéroatome tel que oxygène ou soufre, R₁₀ désigne un atome d'hydrogène, un groupement phényle ou benzyle, R₈ désigne un atome d'hydrogène, un groupement alkyle inférieur ou carboxyle, R₉ désigne un atome d'hydrogène, un groupement alkyle inférieur, un groupement -CH₂-COOH, phényle ou benzyle ;
Dans la formule précitée un radical alkyle inférieur désigne de préférence un groupement ayant 1 à 4 atomes de carbone et en particulier, méthyle et éthyle.

Les polymères fixants anioniques à groupements carboxyliques préférés selon l'invention sont :
A) les homo- ou copolymères d'acide acrylique ou méthacrylique ou leurs sels et en particulier les produits commercialisés sous les dénominations VERSICOL E ou K par la société ALLIED COLLOID et ULTRAHOLD par la société BASF. Les copolymères d'acide acrylique et d'acrylamide commercialisés sous la forme de leur sel de sodium sous les dénominations RETEN 421, 423 ou 425 par la Société HERCULES, les sels de sodium des acides polyhydroxycarboxyliques.
B) les copolymères des acides acrylique ou méthacrylique avec un monomère monoéthylénique tel que l'éthylène, le styrène, les esters vinyliques, les esters d'acide acrylique ou méthacrylique. Ces copolymères peuvent être greffés sur un polyalkylène glycol tel que le polyéthylène glycol et éventuellement réticulés. De tels polymères sont décrits en particulier dans le brevet français 1.222.944 et la demande allemande 2.330.956. On peut notamment citer les copolymères comportant dans leur chaîne un motif acrylamide éventuellement N-alkylé et/ou hydroxyalkylé tels que décrits notamment dans les demandes de brevets luxembourgeois 75370 et 75371 ou proposés sous la dénomination QUADRAMER par la Société AMERICAN CYANAMID. On peut également citer les copolymères d'acide acrylique et de méthacrylate d'alkyle en C₁-C₄ et les terpolymères de vinylpyrrolidone, d'acide (méth)acrylique et de (méth)acrylate d'alkyle en C₁-C₂₀ par exemple de lauryle (tel que celui commercialisé par la société ISP sous la dénomination ACRYLIDONE LM), de tertiobutyle (LUVIFLEX VBM 70 commercialisé par BASF) ou de méthyle (STEPANHOLD EXTRA commercialisé par STEPAN) et les terpolyméres acide méthacrylique/ acrylate d'éthyle/ acrylate de tertiobutyle tel que le produit commercialisé sous la dénomination LUVIMER 100 P par la société BASF.
C) les copolymères dérivés d'acide crotonique tels que ceux comportant dans leur chaîne des motifs acétate ou propionate de vinyle et éventuellement d'autres monomères tels que esters allylique ou méthallylique, éther vinylique ou ester vinylique d'un acide carboxylique saturé linéaire ou ramifié à longue chaîne hydrocarbonée tels que ceux comportant au moins 5 atomes de carbone, ces polymères pouvant éventuellement être greffés et réticulés ou encore un ester vinylique, allylique ou méthallylique d'un acide carboxylique α- ou β-cyclique. De tels polymères sont décrits entre autres dans les brevets français 1.222.944, 1.580.545, 2.265.782, 2.265.781, 1.564.110 et 2.439.798. Des produits commerciaux entrant dans cette classe sont les résines 28-29-30, 26-13-14 et 28-13-10 commercialisées par la société NATIONAL STARCH.
D) les copolymères dérivés d'acides ou d'anhydrides carboxyliques monoinsaturés en C4-C8 choisis parmi :
   - les copolymères comprenant (i) un ou plusieurs acides ou anhydrides maiéique, fumarique, itaconique et (ii) au moins un monomère choisis parmi les esters vinyliques, les éthers vinyliques, les halogénures vinyliques, les dérivés phénylvinyliques, l'acide acrylique et ses esters, les fonctions anhydrides de ces copolymères étant éventuellement monoestérifiées ou monoamidifiées ; De tels polymères sont décrits en particulier dans les brevets US 2.047.398, 2.723.248, 2.102.113, le brevet GB 839.805 et notamment ceux commercialisés sous les dénominations GANTREZ AN ou ES, AVANTAGE CP par la société ISP.
   - les copolymères comprenant (i) un ou plusieurs anhydrides maléique, citraconique, itaconique et (ii) un ou plusieurs monomères choisis parmi les esters allyliques ou méthallyliques comportant éventuellement un ou plusieurs groupement acrylamide, méthacrylamide, α-oléfine, esters acryliques ou méthacryliques, acides acrylique ou méthacrylique ou vinylpyrrolidone dans leur chaîne,
   les fonctions anhydrides de ces copolymères étant éventuellement monoestérifiées ou monoamidifiées.
   Ces polymères sont par exemple décrits dans les brevets français 2.350.384 et 2.357.241 de la demanderesse.
E) les polyacrylamides comportant des groupements carboxylates.

Les polymères comprenant les groupements sulfoniques sont des polymères comportant des motifs vinylsulfonique, styrène sulfonique, naphtalène sulfonique ou acrylamido alkylsulfonique.

Ces polymères peuvent être notamment choisis parmi :
- les sels de l'acide polyvinylsulfonique ayant un poids moléculaire moyen en poids compris entre environ 1.000 et 100.000 ainsi que les copolymères avec un comonomère insaturé tel que les acides acrylique ou méthacrylique et leurs esters ainsi que l'acrylamide ou ses dérivés, les éthers vinyliques et la vinylpyrrolidone.
- les sels de l'acide polystyrène sulfonique les sels de sodium ayant un poids moléculaire moyen en poids d'environ 500.000 et d'environ 100.000 commercialisés respectivement sous les dénominations Flexan 500 et Flexan 130 par National Starch. Ces composés sont décrits dans le brevet FR 2.198.719.
- les sels d'acides polyacrylamide sulfoniques ceux mentionnés dans le brevet US 4.128.631 et plus particulièrement l'acide polyacrylamidoéthylpropane sulfonique commercialisé sous la dénomination COSMEDIA POLYMER HSP 1180 par Henkel.

Selon l'invention, les polymères fixants anioniques sont de préférence choisis parmi les copolymères d'acide acrylique tels que les terpolymères acide acrylique / acrytate d'éthyle / N-tertiobutylacrylamide commercialisés notamment sous la dénomination ULTRAHOLD STRONG par la société BASF, les copolymères dérivés d'acide crotonique tels que les terpolymères acétate de vinyle / tertio-butyl benzoate de vinyle *I* acide crotonique et les terpolyméres acide crotonique / acétate de vinyle/néododécanoate de vinyle commercialisés notamment sous la dénomination Résine 28-29-30 par la société NATIONAL STARCH, les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifiés commercialisés par exemple sous la dénomination GANTREZ par la société ISP, les copolymères d'acide méthacrylique et de méthacrylate de méthyle commercialisés sous la dénomination EUDRAGIT L par la société ROHM PHARMA, les copolymères d'acide méthacrylique/ méthacrylate de méthyle / acrylate d'alkyle en C1-C4 / acide acrylique ou méthacrylate d'hydroxyalkyle en C1-C4 commercialisés sous forme de dispersions sous la dénomination AMERHOLD DR 25 par la société AMERCHOL ou sous la dénomination ACUDYNE 255 par la société ROHM & HAAS, les copolymères d'acide méthacrylique et d'acrylate d'éthyle commercialisés sous la dénomination LUVIMER MAEX ou MAE par la société BASF et les copolymères acétate de vinyle/acide crotonique et les copolymères acétate de vinyle/acide crotonique greffés par du polyéthylèneglycol sous la dénomination ARISTOFLEX A par la société BASF.

Les polymères fixants anioniques les plus particulièrement préférés sont choisis parmi les copolymères méthylvinyléther / anhydride maléique mono estérifiés commercialisés sous la dénomination GANTREZ ES 425 par la société ISP, les terpolymères acide acrylique / acrylate d'éthyle / N-tertiobutylacrylamide commercialisés sous la dénomination ULTRAHOLD STRONG par la société BASF, les copolymères d'acide méthacrylique et de méthacrylate de méthyle commercialisés sous la dénomination EUDRAGIT L par la société ROHM PHARMA, les terpolymères acétate de vinyle / tertio-butyl benzoate de vinyle / acide crotonique et les terpolymères acide crotonique / acétate de vinyle / néododécanoate de vinyle commercialisés sous la dénomination Résine 28-29-30 par la société NATIONAL STARCH, les copolymères d'acide méthacrylique et d'acrylate d'éthyle commercialisés sous la dénomination LUVIMER MAEX OU MAE par la société BASF, les terpolymères vinylpyrrolidone / acide acrylique / méthacrylate de lauryle commercialisés sous la dénomination ACRYLIDONE LM par la société ISP.

Les polymères fixants non ioniques utilisables selon la présente invention sont choisis par exemple parmi :
- les homopolymères de vinyllactame tels que la polyvinylpyrrolidone et la polyvinylcaprolactame;
- les copolymères de vinyllactame tels que les copolymères de vinylpyrrolidone et d'acétate de vinyle et les copolymères vinylpyrrolidone / acétate de vinyle / propionate de vinyle ;
- les polyalkyloxazolines telles que les polyéthyloxazolines proposées par la société DOW CHEMICAL sous les dénominations PEOX 50 000, PEOX 200 000 et PEOX 500 000 ;
- les homopolymères d'acétate de vinyle tels que le produit proposé sous le nom de APPRETAN EM par la société HOECHST ou le produit proposé sous le nom de RHODOPAS A 012 par la société RHONE POULENC ;
- les copolymères d'acétate de vinyle et d'ester acrylique tels que le produit proposé sous le nom de RHODOPAS AD 310 de RHONE POULENC ;
- les copolymères d'acétate de vinyle et d'éthylène tels que le produit proposé sous le nom de APPRETAN TV par la société HOECHST ;
- les copolymères d'acétate de vinyle et d'ester maléique par exemple de maléate de dibutyle tels que le produit proposé sous le nom de APPRETAN MB EXTRA par la société HOECHST ;
- les copolymères de polyéthylène et d'anhydride maléique ;
- les homopolymères d'acrytates d'alkyle et les homopolymères de méthacrylates d'alkyle tels que le produit proposé sous la dénomination MICROPEARL RQ 750 par la société MATSUMOTO ou le produit proposé sous la dénomination LUHYDRAN A 848 S par la société BASE ;
- les copolymères d'esters acryliques tels que par exemple les copolymères d'acrylates d'alkyle et de méthacrylates d'alkyles tels que les produits proposés par la société ROHM&HAAS sous les dénominations PRIMAL AC-261 K et EUDRAGIT NE 30 D, par la société BASF sous les dénominations ACRONAL 601, LUHYDRAN LR 8833 ou 8845, par la société HOECHST sous les dénominations APPRETAN N 9213 ou N9212;
- les copolymères d'acrylonitrile et d'un monomère non ionique choisi par exemple parmi le butadiène et les (méth)acrylates d'alkyle ; on peut citer les produits proposés sous les dénominations NIPOL LX 531 B par la société NIPPON ZEON ou ceux proposés sous la dénomination CJ 0601 B par la société ROHM & HAAS ;
- les polyuréthannes tels que les produits proposés sous les dénominations ACRYSOL RM 1020 ou ACRYSOL RM 2020 par la société ROHM & HAAS, les produits URAFLEX XP 401 UZ, URAFLEX XP 402 UZ par la société DSM RESINS;
- les copolymères d'acrylate d'alkyle et d'uréthanne tels que le produit 8538-33 par la société NATIONAL STARCH ;
- les polyamides tels que le produit ESTAPOR LO 11 proposé par la société RHONE POULENC.
- les gommes de guar non ioniques chimiquement modifiées ou non modifiées.
Les gommes de guar non ioniques non modifiées sont par exemple les produits commercialisés sous la dénomination VIDOGUM GH 175 par la société UNIPECTINE et sous la dénomination JAGUAR C par la société MEYHALL.
Les gommes de guar non-ioniques modifiées utilisables selon l'invention sont de préférence modifiées par des groupements hydroxyalkyle en C₁-C₆. On peut mentionner à titre d'exemple, les groupements hydroxyméthyle, hydroxyéthyle, hydroxypropyle et hydroxybutyle.

Ces gommes de guar sont bien connues de l'état de la technique et peuvent par exemple être préparées en faisant réagir des oxydes d'alcènes correspondants, tels que par exemple des oxydes de propylène, avec la gomme de guar de façon à obtenir une gomme de guar modifiée par des groupements hydroxypropyle.

De telles gommes de guar non-ioniques éventuellement modifiées par des groupements hydroxyalkyle sont par exemple commercialisées sous les dénominations commerciales JAGUAR HP8, JAGUAR HP60 et JAGUAR HP120, JAGUAR DC 293 et JAGUAR HP 105 par la société MEYHALL, ou sous la dénomination GALACTASOL 4H4FD2 par la société AQUALON.

Les radicaux alkyle des polymères non ioniques ont de 1 à 6 atomes de carbone sauf mention contraire.

Selon l'invention, on peut également utiliser les polymères fixants anioniques ou non ioniques de type siliconés greffés comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-0 412 704, EP-A-0 412 707, EP-A-0 640 105 et WO 95/00578, EP-A-0582 152 et WO 93/23009 et les brevets US 4,693,935, US 4,728,571 et US 4,972,037.
De tels polymères sont par exemple les copolymères susceptibles d'être obtenus par polymérisation radicalaire à partir du mélange de monomères constitué par:
a) 50 à 90% en poids d'acrylate de tertiobutyle;
b) 0 à 40% en poids d'acide acrylique;
c) 5 à 40% en poids de macromère siliconé de formule: 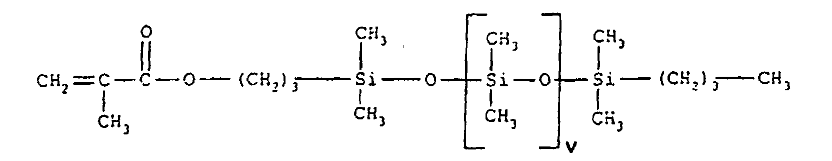
avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon la présente invention, les polymères fixants sont de préférence des polymères anioniques.

Les polymères fixants anioniques peuvent être si nécessaire neutralisés partiellement ou totalement. Les agents de neutralisation sont par exemple la soude, la potasse, l'amino-2 méthyl-2 propanol-1, la monoéthanolamine, la triéthanolamine ou la triisopropanolamine, les acides minéraux ou organiques tels que l'acide chlorhydrique ou l'acide citrique.

Le ou les polymères fixants sont par exemple présents dans des concentrations comprises entre 0,05% et 20% en poids, et de préférence dans des concentrations comprises entre 0,1% et 10% en poids par rapport au poids total de la composition.

Le ou les silicones à groupements carboxyliques peuvent être présentes dans des concentrations comprises entre 0,01% et 10% en poids, et de préférence dans des concentrations comprises entre 0,05% et 5% en poids et encore plus particulièrement de 0,1 à 2% en poids par rapport au poids total de la composition.

Le milieu cosmétiquement acceptable comprend généralement de l'eau, un ou plusieurs solvants cosmétiquement acceptables ou un mélange d'eau et de solvant(s) cosmétiquement acceptable(s).

De préférence, le milieu cosmétiquement acceptable comprend un ou plusieurs solvants cosmétiquement acceptables.

La concentration en solvant cosmétiquement acceptable est généralement supérieure à 20% en poids par rapport au poids total de la composition.

La concentration en eau est généralement inférieure à 80% en poids par rapport au poids total de la composition.

Les solvants cosmétiquement acceptables sont par exemple des monoalcools, des polyalcools, des éthers de glycol ou des esters d'acides gras, qui peuvent être utilisés seuls ou en mélange. Ces solvants sont de préférence des alcools en C₁-C₆.

Parmi ces alcools, on peut citer l'éthanol, l'isopropanol, les polyalcools tels que le diéthylèneglycol, les éthers de glycol dont les monoalkyléthers d'éthylèneglycol, de diéthylèneglycol, de propylèneglycol ou de dipropylèneglycol. L'éthanol est particulièrement préféré.

La composition de l'invention peut également contenir au moins un additif choisi parmi les épaississants, les esters d'acides gras, les esters d'acides gras et de glycérol, les silicones volatiles ou non volatiles, solubles ou insolubles dans la composition, les tensioactifs, les parfums, les conservateurs, les filtres solaires, les protéines, les vitamines, les céramides, les pseudocéramides, les polymères, les huiles végétales, animales, minérales ou synthétiques et tout autre additif classiquement utilisé dans les compositions cosmétiques pour les fibres kératiniques.

Plus particulièrement, la composition comprend également au moins un polymère cationique.

Ces additifs sont présents dans la composition selon l'invention dans des proportions pouvant aller de 0 à 20% en poids par rapport au poids total de la composition. La quantité précise de chaque additif est fonction de sa nature et est déterminée facilement par l'homme de l'art.

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention peuvent se présenter sous forme de lait, de crème, de lotion épaissie ou non.

Les compositions selon l'invention peuvent être utilisées comme produits rincés et de préférence comme produits non-rincés notamment pour le traitement des cheveux, le maintien de la coiffure ou la mise en forme des fibres kératiniques telles que les cheveux.

L'invention a également pour objet l'utilisation d'une composition telle que définie ci-dessus, comme, ou pour la fabrication, de composition de soin, de coiffage ou de fixation des cheveux.

Elles sont plus particulièrement des produits de coiffage tels que des compositions de fixation (laques) et de coiffage. Les lotions peuvent être conditionnées sous diverses formes notamment dans des vaporisateurs, des flacons pompes ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour la fixation ou le traitement des cheveux.

Lorsque la composition selon l'invention est conditionnée sous forme d'aérosol en vue d'obtenir une laque ou une mousse aérosol, elle comprend au moins un agent propulseur qui peut être choisi parmi les hydrocarbures volatils tels que le n-butane, le propane, l'isobutane, le pentane, un hydrocarbure chloré et/ou fluoré et leurs mélanges. On peut également utiliser en tant qu'agent propulseur le gaz carbonique, le protoxyde d'azote, le diméthyléther, l'azote, l'air comprimé et leurs mélanges.

Selon l'invention, la concentration en propulseur(s) est généralement comprise entre 5 et 90% en poids par rapport au poids total de la composition et de préférence entre 10 et 70% en poids. Les compositions pressurisées sous forme de mousse aérosol comprennent de préférence de 5 à 30% en poids d'agent propulseur par rapport au poids total de la composition.

L'invention a encore pour objet un procédé de traitement cosmétique des fibres kératiniques telles que les cheveux consistant à appliquer sur celles-ci une composition telle que définie précédemment.

La préparation des compositions selon l'invention est réalisée selon des méthodes bien connues de l'état de la technique. En particulier, les ingrédients sont mélangés puis conditionnés dans un récipient approprié selon l'utilisation envisagée.

L'invention va être maintenant plus complètement illustrée à l'aide des exemples suivants qui ne sauraient être considérés comme la limitant aux modes de réalisation décrits. (Dans ce qui suit MA signifie Matière Active).

### EXEMPLE 1

On a préparé trois laques de composition suivante:

| **FORMULATION TESTEE** | **A (Invention)** | **B (Comparatif)** | **C (Comparatif)** |
|---|---|---|---|
| **Silicone carboxylique**^{***1**} | 1g | ― | ― |
| **Silicone oxyalkylénée *3** | ― | 1g | ― |
| **Polymère anionique**^{***2**} | 4g | 4g | 4 g |
| **Ethanol qsp** | 100g | 100 g | 100g |

| | | | |
|---|---|---|---|
| *1- Polydiméthylsiloxane à groupements undécyléniques ( Huile M642 commercialisé par la société WACKER) | | | |
| *2- Terpolymère acide acrylique / acrylate d'éthyle / n-tertio butylacrylamide commercialisé sous la dénomination ULTRAHOLD STRONG par la société BASF | | | |
| *3- Polydiméthylsiloxane à groupements oxyalkylène ( DOW CORNING 190 FLUID commercialisé par la société DOW CORNING ) | | | |

### Schéma de pressurisation :

| | |
|---|---|
| Composition ci-dessus | 65 g |
| Diméthyléther | 35 g |

On introduit 65 g de la composition ci-dessus dans un récipient aérosol, on sertit une valve, puis on introduit le diméthyléther.

On a appliqué chacune de ces compositions sur des mèches de cheveux lavés et séchés.

On a ensuite comparé à l'aide d'un test d'évaluation sensorielle, le démêlage et la douceur des cheveux traités par ces laques.

Le test utilisé a pour objet le classement, par un jury constitué de 12 experts, d'une série de 3 mèches en fonction croissante ou décroissante de l'efficacité du démêlage (facilité de passage du peigne) et de la douceur. Les 3 mèches sont présentées simultanément à l'expert. On lui demande de les classer de la plus facile à démêler à la plus difficile et de la plus douce à la moins douce. L'analyse statistique des résultats est effectuée à l'aide des tables de A. KRAMER (Food Technology 17 - (12), 124 - 125 1963).

Les résultats sont consignés dans les tableaux I et II donnés ci-dessous.

### Démêlage

**Tableau I**

| EXPERTS LAQUES | 1 | 2 | 3 | 4 | 5 | 6 | 7 | 8 | 9 | 10 | 11 | 12 | SOMME DES RANGS |
|---|---|---|---|---|---|---|---|---|---|---|---|---|---|
| A | 1 | 1 | 1 | 1 | 1 | 1 | 1 | 1 | 1 | 1 | 1 | 1 | 12 |
| B | 2 | 2 | 2 | 2 | 2 | 2 | 2 | 2 | 2 | 2 | 2 | 2 | 24 |
| C | 3 | 3 | 3 | 3 | 3 | 3 | 3 | 3 | 3 | 3 | 3 | 3 | 36 |

### Douceur

**Tableau II**

| EXPERTS LAQUES | 1 | 2 | 3 | 4 | 5 | 6 | 7 | 8 | 9 | 10 | 11 | 12 | SOMME DES RANGS |
|---|---|---|---|---|---|---|---|---|---|---|---|---|---|
| A | 1 | 1 | 1 | 1 | 2 | 1 | 1 | 1 | 1 | 2 | 1 | 1 | 14 |
| B | 2 | 2 | 2 | 2 | 1 | 2 | 2 | 2 | 2 | 1 | 2 | 2 | 22 |
| C | 3 | 3 | 3 | 3 | 3 | 3 | 3 | 3 | 3 | 3 | 3 | 3 | 36 |

Selon les tables de KRAMER, si la somme des rang d'une composition est à l'extérieur de l'intervalle 18-30 ( 3 échantillons - 12 experts), cette composition est significativement différente des autres compositions.

La composition A selon l'invention présente donc des propriétés de démêlage et de douceur supérieures à celles des compositions B et C.

### EXEMPLE 2 (Comparatif)

On a préparé trois laques non conformes à l'invention de composition suivante :

| **FORMULATION TESTEE** | **A (Comparatif)** | **B (Comparatif)** | **C (Comparatif)** |
|---|---|---|---|
| **Silicone carboxylique**^{***1**} | 1 g | ― | ― |
| **Silicone oxyalkylénée *3** | ― | 1g | ― |
| **Polymère cationique**^{***2**} | 5,7 gMA | 5,7 gMA | 5,7 gMA |
| **Ethanol qsp** | 100 g | 100 g | 100g |

| | | | |
|---|---|---|---|
| *1- Polydiméthylsiloxane à groupements undécyléniques ( Huile M642 WACKER) | | | |
| *2- Terpolymère vinylcaprolactame / vinylpyrrolidone / méthacrylate de diméthylaminoéthyle commercialisé sous la dénomination GAFFIX VC 713 par la société ISP | | | |
| *3 - Polydiméthylsiloxane à groupements oxyalkyléne ( DOW CORNING 190 FLUID commercialisé par la société DOW CORNING) | | | |

### Schéma de pressurisation :

| | |
|---|---|
| Composition ci-dessus | 65 g |
| Diméthyléther | 35 g |

On introduit 65 g de la composition ci-dessus dans un récipient aérosol, on sertit une valve, puis on introduit le diméthyléther.

On a appliqué chacune de ces compositions sur des mèches de cheveux lavés et séchés.

On a ensuite comparé à l'aide d'un test d'évaluation sensorielle la douceur des cheveux traités par ces laques.

On a donc demandé à un panel de 10 experts de classer les mèches traitées avec chaque composition selon le critère de douceur.

La composition A contenant la silicone à groupement carboxylique et un polymère cationique présente des propriétés de douceur inférieures à celles de la composition B contenant une silicone oxyalkylénée et du même niveau que celles de la composition ne contenant que le polymère cationique.
La silicone carboxylique ne permet donc pas d'améliorer la douceur des cheveux traités avec un polymère cationique.

### EXEMPLE 3

On a préparé deux laques de composition suivante :

| **FORMULATION TESTEE** | **A (invention)** | **B (Comparatif)** |
|---|---|---|
| **Silicone carboxylique**^{***1**} | 1 g | ― |
| **Silicone oxyalkylénée*3** | ― | 1 g |
| **Polymère non ionique**^{***2**} | 4 gMA | 4 gMA |
| **Ethanol qsp** | 100 g | 100 g |

| | | |
|---|---|---|
| *1- Polydiméthylsiloxane à groupements undécyléniques ( Huile M642 commercialisée par la société WACKER) | | |
| *2-Polyvinylcaprolactame | | |
| *3-Polydiméthylsiloxane à groupements oxyalkylénés (DOW CORNING 190 FLUID) commercialisé par la société DOW CORNING | | |

### Schéma de pressurisation :

| | |
|---|---|
| Composition ci-dessus | 65 g |
| Diméthyléther | 35 g |

On introduit 65 g de la composition ci-dessus dans un récipient aérosol, on sertit une valve, puis on introduit le diméthyléther.

On a appliqué chacune de ces compositions sur deux demi-têtes de cheveux lavés et séchés.
Puis, on a demandé à un panel de 10 experts d'évaluer le démêlage et la douceur et le pouvoir fixant des mèches traitées avec chaque composition.

La composition A (invention) présente des propriétés de douceur et un pouvoir fixant supérieurs à ceux de la composition B.

### EXEMPLE 4

On a préparé une composition de spray fixant conditionnée dans un récipient aérosol de composition suivante :
- Terpolymère acide acrylique / acrylate d'éthyle / n-tertio butylacrylamide commercialisé sous la dénomination ULTRAHOLD STRONG par la société BASF 4g
- Amino-2 méthyl-2 propanol-1 qs neutralisation à 100% du terpolymère
- Polydiméthylsiloxane à groupements undécyléniques commercialisé sous la dénomination Huile M 642 par la société WACKER 1 g
- Ethanol qsp 100 g

### Schéma de pressurisation :

| | |
|---|---|
| Composition ci-dessus | 65 g |
| Diméthyléther | 35 g |

On introduit 65 g de la composition ci-dessus dans un récipient aérosol, on sertit une valve, puis on introduit le diméthyléther.

Les cheveux traités avec la composition selon l'invention présentent de bonnes propriétés de toucher, de douceur et de démêlage.

### EXEMPLE 5

On a préparé une composition de spray fixant conditionnée dans un récipient aérosol de composition suivante :
- Terpolymère acide acrylique / acrylate d'éthyle / n-tertio butylacrylamide commercialisé sous la dénomination ULTRAHOLD STRONG par la société BASF 2,3 g
- Amino-2 méthyl-2 propanol-1 qs neutralisation à 100% du terpolymére
- Terpolymère vinylcaprolactame / vinylpyrrolidone / méthacrylate de diméthylaminoéthyle commercialisé sous la dénomination GAFFIX VC 713 par la société ISP 3,8 gMA
- Polydiméthylsiloxane à groupements undécyléniques commercialisé sous la dénomination Huile M 642 par la société WACKER 0,8 g
- Ethanol qsp 100 g

### Schéma de pressurisation :

| | |
|---|---|
| Composition ci-dessus | 65 g |
| Diméthyléther | 35 g |

On introduit 65 g de la composition ci-dessus dans un récipient aérosol, on sertit une valve, puis on introduit le diméthyléther.

### EXEMPLE 6

On a préparé une composition de spray fixant conditionnée dans un flacon-pompe de composition suivante :
- Terpolymère acétate de vinyle /p-tertiobutyl benzoate de vinyle/ acide crotonique (polymère fixant) 5 g
- Amino-2 méthyl-2 propanol-1
   (qs neutralisation à 100% du polymère fixant)
- Polydiméthylsiloxane à groupements undécylèniques commercialisé sous la dénomination Huile M 642 par la société WACKER 1 g
- Ethanol qsp 100 g

### EXEMPLE 7

On a préparé une composition de spray fixant conditionnée dans un flacon-pompe de composition suivante :
- Polyvinylcaprolactame 6 gMA
- Polydiméthylsiloxane à groupements undécyléniques commercialisé sous la dénomination Huile M 642 par la société WACKER 1,2 g
- Eau 37,8 g
- Ethanol qsp 100 g

### EXEMPLE 8

On a préparé une mousse de coiffage de composition suivante :
- Terpolymère acide acrylique / acrylate d'éthyle / n-tertio butylacrylamide commercialisé sous la dénomination ULTRAHOLD STRONG par la société BASF (polymère fixant) 2 g
- Amino-2 méthyl-2 propanol-1
   (qs neutralisation à 100% du polymère fixant)
- Polydiméthylsiloxane à groupements undécyléniques commercialisé sous la dénomination Huile M 642 par la société WACKER 1 g
- Polyvinylcaprolactame 1 gMA
- Mélange d'alcools cétylstéarylique et cétylstéarylique oxyéthyléné à 5 moles d'oxyde d'éthylène commercialisé sous la dénomination Polawax A 31 par la société CRODA 0,5 g
- Ethanol 25 g
- Eau qsp 100 g

### Conditionnement en aérosol :

90g de la composition ci-dessus sont conditionnés dans un récipient aérosol en présence de 10g d'un mélange ternaire de n-butane, isobutane et propane, (23/55/22), commercialisé sous la dénomination de "AEROGAZ 3,2 N" par la société ELF AQUITAINE.

La composition est appliquée sur des cheveux lavés et essorés. Elle conduit à un bon maintien de la coiffure et à de bonnes propriétés de démêlage et de douceur.

## Revendications

1. Composition cosmétique, **caractérisée en ce qu'**elle comprend, dans un milieu cosmétiquement acceptable, au moins un polymère fixant anionique ou non ionique et au moins une silicone comprenant au moins une fonction acide carboxylique, qui ne résulte pas de la polymérisation d'un ou plusieurs monomères anioniques à insaturation éthylénique.

2. Composition selon la revendication précédente, **caractérisée en ce que** le ou les polymères fixants sont présents dans des concentrations comprises entre 0,05 et 20% en poids, et de préférence dans des concentrations comprises entre 0,1 et 10%, en poids par rapport au poids total de la composition.

3. Composition selon l'une des revendications précédentes, **caractérisée en ce que** la silicone est présente dans des concentrations comprises entre 0,01% et 10% en poids par rapport au poids total de la composition, de préférence dans des concentrations comprises entre 0,05% et 5% en poids et plus particulièrement entre 0,1 et 2% en poids.

4. Composition selon l'une quelconque des revendications 1 à 3, **caractérisée en ce que** la silicone comprenant au moins un groupement acide carboxylique est un organopolysiloxane comprenant au moins un motif (I) : 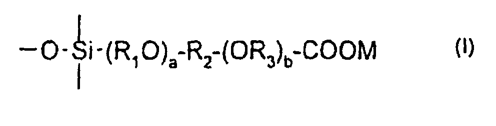 dans lequel R₁ et R₃ désignent indépendamment un radical alkylène linéaire ou ramifié ayant de 2 à 20 atomes de carbone, R₂ désigne un radical alkylène linéaire ou ramifié ayant de 1 à 50 atomes de carbone pouvant comprendre un groupement hydroxyle,
a représente 0 ou 1 et b est un nombre allant de 0 à 200, M désigne hydrogène, un métal alcalin ou alcalino-terreux, NH₄, un groupement ammonium quaternaire.

5. Composition selon la revendication 4, **caractérisée en ce que** les organopolysiloxanes sont choisis parmi les composés de formule (II) : 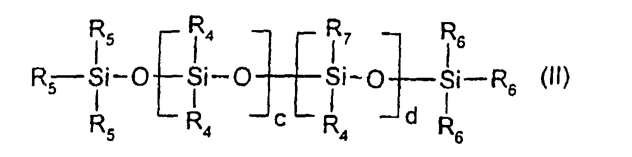 dans laquelle les radicaux R₄ sont identiques ou différents les uns des autres et sont choisis parmi un radical alkyle en C₁-C₂₂, linéaire ou ramifié, un radical alcoxy en C₁-C₂₂ et un radical phényle,
les radicaux R₅, R₆, R₇ sont identiques ou différents les uns des autres et sont choisis parmi un radical alkyle en C₁-C₂₂, linéaire ou ramifié, un radical alcoxy en C₁-C₂₂ et un radical phényle et un radical -(R₁O)ₐ-R₂-(OR₃)_{b}-COOM,
au moins l'un des radicaux R5, R6, R7 étant un radical -(R₁O)ₐ-R₂-(OR₃)_{b}-COOM, les radicaux R₁, R₂, R₃, a, b et M ayant la même signification que ci-dessus,
c et d sont des nombres allant de 0 à 1000, la somme c+d allant de préférence de 2 à 1000.

6. Composition selon l'une quelconque des revendications précédentes, **caractérisée par le fait que** le polymère fixant anionique est choisi parmi :
- les copolymères d'acide acrylique tels que les terpolymères acide acrylique/acrylate d'éthyle/N-tertiobutylacrylamide ;
- les copolymères dérivés d'acide crotonique tels que les terpolymères acétate de vinyle / tertio-butyl benzoate de vinyle / acide crotonique et les terpolymères acide crotonique/acétate de vinyle/néododécanoate de vinyle ;
- les polymères dérivés d'acides ou d'anhydrides maléique, fumarique, itaconique avec des esters vinyliques, des éthers vinyliques, des halogénures vinyliques, des dérivés phénylvinyliques, l'acide acrylique et ses esters tels que les copolymères méthylvinyléther/anhydride maléique mono estérifié ;
- les copolymères d'acide méthacrylique et de méthacrylate de méthyle ;
- les copolymères d'acide méthacrylique et d'acrylate d'éthyle;
- les copolymères acétate de vinyle/acide crotonique ;
- les terpolymères acétate de vinyle/acide crotonique/polyéthylèneglycol ;
- les copolymères d'acide méthacrylique/ méthacrylate de méthyle / acrylate d'alkyle en C1-C4 / acide acrylique ou méthacrylate d'hydroxyalkyle en C1-C4.

7. Composition selon l'une des revendications 1 à 5, **caractérisé par le fait que** le polymère fixant non ionique est choisi parmi :
- les homopolymères de vinyllactame ;
- les copolymères de vinyllactame tels que les copolymères de vinylpyrrolidone et d'acétate de vinyle ;
- les polyalkyloxazolines ;
- les homopolymères d'acétate de vinyle ;
- les copolymères d'acétate de vinyle et d'ester acrylique ;
- les copolymères d'acétate de vinyle et d'éthylène ;
- les copolymères d'acétate de vinyle et d'ester maléique ;
- les copolymères de polyéthylène et d'anhydride maléique ;
- les homopolymères d'acrylates d'alkyle et les homopolymères de méthacrylates d'alkyle ;
- les copolymères d'esters acryliques tels que par exemple les copolymères d'acrylates d'alkyle et de méthacrylates d'alkyles ;
- les copolymères d'acrylonitrile et d'un monomère non ionique choisi par exemple parmi le butadiène et les (méth)acrylates d'alkyle ;
- les copolymères d'acrylate d'alkyle et d'uréthanne.

8. Composition selon l'une quelconque des revendications précédentes, **caractérisée en ce que** le milieu cosmétiquement acceptable comprend de l'eau, un ou plusieurs solvants cosmétiquement acceptables ou un mélange d'eau et de solvant(s) cosmétiquement acceptable(s).

9. Procédé de traitement cosmétique des fibres kératiniques telles que les cheveux consistant à appliquer sur celles-ci une composition telle que définie à l'une quelconque des revendications précédentes.

10. Utilisation d'une composition telle que définie dans l'une quelconque des revendications 1 à 8, comme, ou pour la fabrication, de composition de soin, de coiffage ou de fixation des cheveux.

## Claims

1. Cosmetic composition, **characterized in that** it comprises, in a cosmetically acceptable medium, at least one anionic or nonionic fixing polymer and at least one silicone comprising at least one carboxylic acid function which does not result from the polymerization of one or more anionic monomers containing ethylenic unsaturation.

2. Composition according to the preceding claim, **characterized in that** the fixing polymer(s) is (are) present in concentrations of between 0.05 and 20% by weight, and preferably in concentrations of between 0.1 and 10% by weight, relative to the total weight of the composition.

3. Composition according to either of the preceding claims, **characterized in that** the silicone is present in concentrations of between 0.01% and 10% by weight relative to the total weight of the composition, preferably in concentrations of between 0.05% and 5% by weight and more particularly between 0.1 and 2% by weight.

4. Composition according to any one of Claims 1 to 3, **characterized in that** the silicone comprising at least one carboxylic acid group is an organopolysiloxane comprising at least one unit (I) : 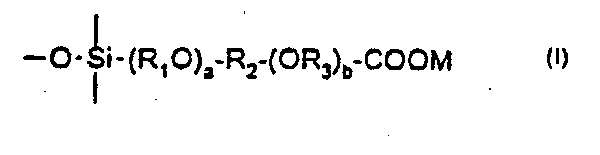 in which R₁ and R₃ independently denote a linear or branched alkylene radical containing from 2 to 20 carbon atoms and R₂ denotes a linear or branched alkylene radical containing from 1 to 50 carbon atoms which can comprise a hydroxyl group,
a represents 0 or 1, b is a number ranging from 0 to 200 and M denotes hydrogen, an alkali metal or alkaline-earth metal, NH₄ or a quaternary ammonium group.

5. Composition according to Claim 4, **characterized in that** the organopolysiloxanes are chosen from the compounds of formula (II) : 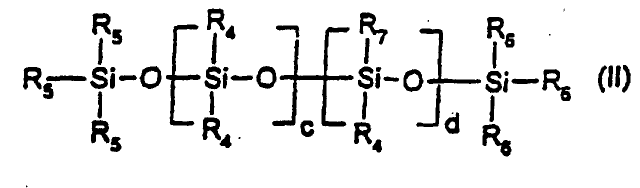 in which the radicals R₄ are identical to or different from each other and are chosen from a linear or branched C₁-C₂₂ alkyl radical, a C₁-C₂₂ alkoxy radical and a phenyl radical,
the radicals R₅, R₆ and R₇ are identical to or different from each other and are chosen from a linear or branched C₁-C₂₂ alkyl radical, a C₁-C₂₂ alkoxy radical, a phenyl radical and a radical -(R₁O)ₐ-R₂-(OR₃)_{b}-COOM,
at least one of the radicals R₅, R₆ and R₇ being a radical -(R₁O)ₐ-R₂-(OR₃)_{b}-COOM,
the radicals R₁, R₂, R₃, a, b and M having the same meaning as above,
c and d are integers ranging from 0 to 1 000, the sum c+d preferably ranging from 2 to 1 000.

6. Composition according to any one of the preceding claims, **characterized in that** the anionic fixing polymer is chosen from:
- acrylic acid copolymers such as acrylic acid/ethyl acrylate/N-tert-butylacrylamide terpolymers;
- copolymers derived from crotonic acid, such as vinyl acetate/vinyl tert-butylbenzoate/crotonic acid terpolymers and crotonic acid/vinyl acetate/vinyl neododecanoate terpolymers;
- polymers derived from maleic, fumaric or itaconic acids or anhydrides with vinyl esters, vinyl ethers, vinyl halides, phenylvinyl derivatives or acrylic acid and its esters, such as methyl vinyl ether/maleic anhydride monoesterified copolymers;
- copolymers of methacrylic acid and of methyl methacrylate;
- copolymers of methacrylic acid and of ethyl acrylate;
- vinyl acetate/crotonic acid copolymers;
- vinyl acetate/crotonic acid/polyethylene glycol terpolymers;
- methacrylic acid/methyl methacrylate/C₁-C₄ alkyl acrylate/acrylic acid or C₁-C₄ hydroxyalkyl methacrylate copolymers.

7. Composition according to one of Claims 1 to 5, **characterized in that** the nonionic fixing polymer is chosen from:
- vinyllactam homopolymers;
- vinyllactam copolymers such as copolymers of vinylpyrrolidone and of vinyl acetate;
- polyalkyloxazolines;
- vinyl acetate homopolymers;
- copolymers of vinyl acetate and of acrylic ester;
- copolymers of vinyl acetate and of ethylene;
- copolymers of vinyl acetate and of maleic ester;
- copolymers of polyethylene and of maleic anhydride;
- alkyl acrylate homopolymers and alkyl methacrylate homopolymers;
- acrylic ester copolymers such as, for example, copolymers of alkyl acrylates and of alkyl methacrylates;
- copolymers of acrylonitrile and of a nonionic monomer chosen, for example, from butadiene and alkyl (meth)acrylates;
- copolymers of alkyl acrylate and of urethane.

8. Composition according to any one of the preceding claims, **characterized in that** the cosmetically acceptable medium comprises water, one or more cosmetically acceptable solvents or a mixture of water and cosmetically acceptable solvent(s).

9. Process for the cosmetic treatment of keratin fibres such as the hair, which consists in applying a composition as defined in any one of the preceding claims to these fibres.

10. Use of a composition as defined in any one of Claims 1 to 8, as, or for the manufacture of, a care, styling or fixing composition for the hair.

## Patentansprüche

1. Kosmetische Zusammensetzung, **dadurch gekennzeichnet, daß** sie in einem kosmetisch akzeptablen Medium mindestens ein anionisches oder nichtionisches Polymer und mindestens ein Silicon enthält, das mindestens eine Carboxyfunktion aufweist, die nicht aus der Polymerisation eines oder mehrerer anionischer Monomere mit ethylenischer Doppelbindung resultiert.

2. Zusammensetzung nach dem vorhergehenden Anspruch, **dadurch gekennzeichnet, daß** das fixierende Polymer oder die fixierenden Polymere in Konzentrationen im Bereich von 0,05 bis 20 Gew.-%, und vorzugsweise im Bereich von 0,1 bis 10 Gew.-%, bezogen auf das Gesamtgewicht der Zusammensetzung, vorliegen.

3. Zusammensetzung nach einem der vorhergehenden Ansprüche, **dadurch gekennzeichnet, daß** das Silicon in Konzentrationen im Bereich von 0,01 bis 10 Gew.-%, vorzugsweise im Bereich von 0,05 bis 5 Gew.-% und insbesondere 0,1 bis 2 Gew.-%, bezogen auf das Gesamtgewicht der Zusammensetzung, vorliegt.

4. Zusammensetzung nach einem der Ansprüche 1 bis 3, **dadurch gekennzeichnet, daß** das Silicon mit mindestens einer Carbonsäuregruppe ein Organopolysiloxan ist, das mindestens eine Einheit (I): 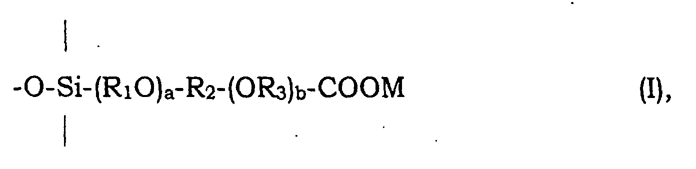 enthält, wobei die Gruppen R₁ und R₃ unabhängig voneinander geradkettige oder verzweigte Alkylengruppen mit 2 bis 20 Kohlenstoffatomen bedeuten und die Gruppe R₂ eine geradkettige oder verzweigte Alkylengruppe mit 1 bis 50 Kohlenstoffatomen ist, die eine Hydroxygruppe enthalten kann, a 0 oder 1 bedeutet und b eine Zahl im Bereich von 0 bis 200 ist, und M Wasserstoff, ein Alkalimetall, ein Erdalkalimetall, NH₄ oder eine quartäre Ammoniumgruppe bedeutet.

5. Zusammensetzung nach Anspruch 4, **dadurch gekennzeichnet, daß** die Organopolysiloxane unter den Verbindungen der folgenden Formel (II) ausgewählt sind: 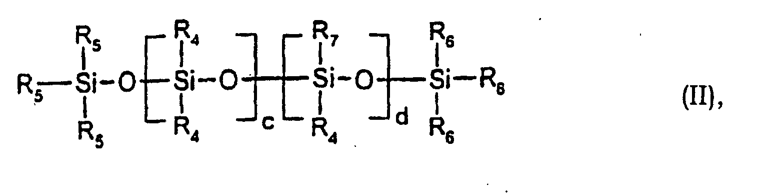 worin:
- die Gruppen R₄, die identisch oder voneinander verschieden sind, unter den geradkettigen oder verzweigten C₁₋₂₂-Alkylgruppen, C₁₋₂₂-Alkoxygruppen und Phenyl ausgewählt sind,
- die Gruppen R₅, R₆ und R₇, die identisch oder voneinander verschieden sind, unter den geradkettigen oder verzweigten C₁₋₂₂-Alkylgruppen, C₁₋₂₂-Alkoxygruppen, Phenyl und den Gruppen -(R₁O)ₐ-R₂-(OR₃)_{b}-COOM ausgewählt sind,
- wobei mindestens eine der Gruppen R₅, R₆ und R₇ eine Gruppe -(R₁O)ₐ-R₂-(OR₃)_{b}-COOM ist und R₁, R₂, R₃, a, b und M die oben angegebenen Bedeutungen aufweisen,
- c und d Zahlen von 0 bis 1000 bedeuten, wobei die Summe c + d vorzugsweise im Bereich von 2 bis 1000 liegt.

6. Zusammensetzung nach einem der vorhergehenden Ansprüche, **dadurch gekennzeichnet, daß** das anionische fixierende Polymer ausgewählt ist unter:
- den Acrylsäure-Copolymeren, beispielsweise den Acrylsäure/Ethylacrylat/N- t-Butylacrylamid-Terpolymeren;
- den Copolymeren, die von Crotonsäure abgeleitet sind, beispielsweise den Vinylacetat/Vinyl-t-Butylbenzoat/Crotonsäure-Terpolymeren und den Crotonsäure/Vinylacetat/Vinylneododecanoat-Terpolymeren;
- den Polymeren, die von Maleinsäure, Fumarsäure, Itaconsäure oder deren Anhydriden mit Vinylestern, Vinylethern, Vinylhalogeniden, Phenylvinylderivaten und Acrylsäure und ihren Estern abgeleitet sind, beispielsweise den Copolymeren von Methylvinylether und einfach verestertem Maleinsäureanhydrid;
- den Copolymeren von Methacrylsäure und Methylmethacrylat;
- den Copolymeren von Methacrylsäure und Ethylacrylat;
- den Copolymeren von Vinylacetat und Crotonsäure;
- den Vinylacetat/Crotonsäure/Polyethylenglykol-Terpolymeren;
- den Terpolymeren Methacrylsäure/Methylmethacrylat/C₁₋₄-Alkylacrylat/Acrylsäure oder C₁₋₄-Hydroxyalkylmethacrylat.

7. Zusammensetzung nach einem der Ansprüche 1 bis 5, **dadurch gekennzeichnet, daß** das nichtionische fixierende Polymer ausgewählt ist unter:
- den Vinyllactam-Homopolymeren;
- den Vinyllactam-Copolymeren, wie Copolymeren von Vinylpyrrolidon und Vinylacetat;
- den Polyalkyloxazolinen;
- den Homopolymeren von Vinylacetat;
- den Copolymeren von Vinylacetat und Acrylestern;
- den Copolymeren von Vinylacetat und Ethylen;
- den Copolymeren von Vinylacetat und Maleinsäureestern;
- den Copolymeren von Polyethylen und Maleinsäureanhydrid;
- den Homopolymeren von Alkylacrylaten und den Homopolymeren von Alkylmethacrylaten;
- den Acrylester-Copolymeren, wie beispielsweise den Copolymeren von Alkylacrylaten und Alkylmethacrylaten;
- den Copolymeren von Acrylnitril und einem nichtionischen Monomer, das beispielsweise unter Butadien und den Alkyl(meth)-acrylaten ausgewählt ist;
- den Copolymeren von Alkylacrylat und Urethanen.

8. Zusammensetzung nach einem der vorhergehenden Ansprüche, **dadurch gekennzeichnet, daß** das kosmetisch akzeptable Medium Wasser, ein oder mehrere kosmetisch akzeptable Lösungsmittel oder Gemische von Wasser und einem oder mehreren kosmetisch akzeptablen Lösungsmitteln enthält.

9. Verfahren zur kosmetischen Behandlung von Keratinsubstanzen, wie dem Haar, das darin besteht, auf die Keratinsubstanzen eine Zusammensetzung nach einem der vorhergehenden Ansprüche aufzubringen.

10. Verwendung einer Zusammensetzung nach einem der Ansprüche 1 bis 8 Zusammensetzung zur Pflege, zum Frisieren oder zum Fixieren der Haare oder zur Herstellung einer solchen Zusammensetzung.
